Europäisches Patentamt

(19) European Patent Office

Office européen des brevets

(11) EP 0 950 391 A1

(12) EUROPEAN PATENT APPLICATION

(43) Date of publication:
20.10.1999 Bulletin 1999/42

(51) Int. Cl.$^6$: A61K 7/00

(21) Application number: 99106278.7

(22) Date of filing: 16.04.1999

(84) Designated Contracting States:
AT BE CH CY DE DK ES FI FR GB GR IE IT LI LU MC NL PT SE
Designated Extension States:
AL LT LV MK RO SI

(30) Priority: 17.04.1998 JP 10825998
05.03.1999 JP 5898199

(71) Applicant: KAO CORPORATION
Chuo-ku Tokyo (JP)

(72) Inventors:
• Takeuchi, Katsuhiko,
Sumida-ku, Tokyo (JP)

• Kamiya, Tetsuro
Sumida-ku, Tokyo (JP)
• Hagura, Toyoki
Sumida-ku, Tokyo (JP)
• Honda, Miyuki
Sumida-ku, Tokyo (JP)
• Yokohama, Machiko
Ichikaimachi, Haga-gun, Tochigi (JP)

(74) Representative: HOFFMANN - EITLE
Patent- und Rechtsanwälte
Arabellastrasse 4
81925 München (DE)

(54) **Cosmetic sheet comprising at least one powder**

(57) The present invention provides a cosmetic sheet which gives comfortable and refreshed feeling to the skin caused by efficient adherence of a specific powder on the skin. The cosmetic sheet comprises at least one powder selected from a synthetic polymer and a natural mineral and an aqueous medium held together in a fabric sheet. The cosmetic sheet is applied to the human skin and reduces the dynamic friction resistance of the skin area to 95% or less of that before application. The powder has preferably a surface energy of 70 mN/m or less.

EP 0 950 391 A1

## Description

Field of the Invention

The present invention relates to a cosmetic sheet which comprises a fabric sheet and at least one powder held therein. More specifically, the present invention relates to a cosmetic sheet, which is used for cleansing, wiping off or shaving and gives a comfortable feeling by efficiently adhering the powder on human skin. Also a process for preparing the cosmetic sheet is disclosed.

Background art

Heretofore, various wet sheet products, which are used for cleansing or wiping off face, neck, hands or legs, or shaving, have been put to practical use. They include a product in which each sheet is packaged separately, a pocket tissue-type product in which ten or more sheets are packaged as a unit, and a box-type product in which several tens of sheets are packaged together. These products each comprise several components, such as a cosmetic ingredient, a cleansing agent, a refreshing agent, a germicide and a perfume, impregnated into a sheet substrate. These products are used for cleansing skin, giving a cool feeling, or supplying water for shaving.
However conventional products often produce a dry or unpleasant feeling because oil and water are wiped or cleansed off in excess from the skin.

Summary of the invention

The present inventors have found a cosmetic sheet comprising a fabric sheet incorporated with specific powder and an aqueous medium, wherein the powder exist in the vicinity of the sheet surface and they are efficiently transferred and adhered onto the skin to give a refreshed feeling to the skin after application, e.g. wiping off or cleansing.
Accordingly, the present invention provides a cosmetic sheet which comprises at least one powder selected from a synthetic polymer and a natural mineral and an aqueous medium held together in a fabric sheet.
Further, the present invention provides a cosmetic sheet which reduces the dynamic friction resistance of the applied skin area, after drying, to 95% or less of that before use.
Still further, the present invention provides a cosmetic sheet which comprises a fabric sheet, at least one powder having surface energy of 70 mN/m or less and an aqueous medium incorporated together in the sheet.
The present invention also relates to a method for reducing the dynamic friction resistance of the skin by applying the cosmetic sheet of the present invention so that the powder is transferred and adhered onto the skin.
Further, the present invention relates to a process for preparing a stacked cosmetic sheet product, wherein the process comprises the steps of (A) providing a fabric sheet with a liquid composition containing at least one powder selected from a synthetic polymer and a natural mineral and an aqueous medium so that the liquid composition is held in the fabric sheet, (B) cutting the sheet and (C) stacking the cut sheets.

Detailed description of the invention

[Powder]

The cosmetic sheet of the present invention can be prepared by spraying, dipping or coating a sheet with a liquid composition, which comprises an aqueous medium such as water or water-containing ethanol, and at least one powder selected from synthetic polymers and natural minerals dispersed in the medium, so that the liquid composition is held in the sheet.
Of the powder for use in the present invention, examples of the powder selected from synthetic polymers include organosilicone group-containing polymers such as silicone resin (e.g., KMP-590 and KMP-599 manufactured by Shin-Etsu Co., Ltd., and Tospearl 145 and Tospearl 2000B manufactured by Toshiba Silicone Co., Ltd.); polyorganosilsesquioxane; silicone rubber powder (e.g., KMP-597, KMP-598, and X-52-875 manufactured by Shin-Etsu Co., Ltd., and Trefil 501, Trefil 505, Trefil 506, and Trefil 601 manufactured by Toray Silicone Co., Ltd.); silicone resin/rubber composite powder (e.g., X-52-1139K and X-52-1139G manufactured by Shin-Etsu Co., Ltd.); and polymer fine particles obtained by dispersion-polymerization of a vinyl monomer by using, as a dispersant, a polysiloxane compound having a radical polymerizable group (hereinafter referred to as Polymer Particle X). Further examples include synthetic silica beads; hydrophobic silica powders (e.g., KMP-110 and KMP-105 manufactured by Shin-Etsu Co., Ltd.); nylon resins (e.g., SP-500 manufactured by Toray Co., Ltd.); polystyrene-based resins (e.g., Finepearl manufactured by Sumitomo Chemical Co., Ltd., Techpolymer SB manufactured by Sekisui Chemical Co., Ltd., and Finepowder SGP manufactured by Soken Chemical Co., Ltd.); polyethylene resins (e.g., Flo-beads manufactured by Sumitomo Seika Chemicals Co.,

Ltd.); polymethyl methacryate resins (e.g., Matsumoto Microsphere M manufactured by Matsumoto Yushi-Seiyaku Co., Ltd., Techpolymer MB manufactured by Sekisui Chemical Co., Ltd., and Finepowder MP manufactured by Soken Chemical Co., Ltd.); divinylbenzene-based resins; polyurethane-based resins; benzoguanamine-based resins; melamine resins; phenol resins and fluorine-containing resins. Also usable are those surface-treated with a water-repellant compound containing silicone moiety or fluorine atoms such as a methylhydrogendimethylpolysiloxane and a perfluoroalkyl phosphate diethanol amine salt (e.g., SI-Talc JA46R manufactured by Miyoshi Kasei Inc., and PF-3LL-5 Talc manufactured by Daito Kasei KoGyo Co., Ltd.). Among them, organosilicone group-containing polymers such as said Polymer Particle X and silicone resins are preferable for reduction of dynamic friction resistance and slippery feeling (non-sticky feeling) of the skin, and retention of the powder on the skin.

Examples of the powder selected from natural minerals include talc, sericite, mica, kaolin, red iron oxide, clay, bentonite, silicic acid, silicic acid anhydride, magnesium silicate, magnesium oxide, zinc oxide, titanium oxide, aluminum oxide, aluminum sulfate, alum, calcium sulfate, barium sulfate and magnesium sulfate. Among them, talc is preferable because of excellent slipperiness on the skin.

In the present invention, at least one powder selected from the list above may be used.

These powders are preferably spherical. As the sphericity increases, the dynamic friction resistance decreases to give an improved slippery feeling on the skin. From the standpoint of feeling upon use, a sphericity of 70 or greater is preferred, and that of 90 or greater being particularly preferred. The sphericity referred to above is measured according to the following method.

〈Sphericity〉

An electron microscopic photograph of solid particles is taken by using a scanning electron microscope. 100 projected images of particles out of the photograph, which do not overlap each other, are selected at random. Sphericity is determined by the total number of the images which fulfil either of conditions below:

(1) the images having a true circle,
(2) the images having the size between the circumcircle of each image and the cocentric circle having 90% radius of the circumcircle.

As the diameters of the powders, it is preferable that 60% by weight or more of the whole powder fall in the range of 0.5 to 30 μm to improve the feeling on the skin; and it is particularly preferable that 80% by weight or more of the whole powder fall in the range of 1 to 10 μm. Those particles having diameters of less than 0.5 μm exert little or no effect on the feeling on the skin, sometimes giving an unpleasant feeling such as grating against the skin. Also, the amount of the particles having diameters of more than 30 μm should be reduced. Accordingly the amount of the particles with diameters out of the said prefered range is preferably 40% by weight or less, more preferably 10% by weight or less, based on the whole powder. To give a comfortable feeling to the skin, particles having diameters of 200 μm or greater are substantially eliminated desirably.

In order to give a comfortable feeling without showing white appearance on the skin, the cosmetic sheet of the present invention contains the powders having diameters of 0.5 to 30 μm preferably in amount of 0.1 to 20 g, most preferably 0.5 to 10 g, based on 1 square meter of the sheet.

The ratio of impregnation, i.e. the weight ratio of the aqueous medium containing the powder to the fabric sheet, is preferably from 50 to 500%, most preferably from 100 to 400%, based on the weight of the sheet to improve the characteristic feeling upon use.

[Fabric sheet]

Any of nonwoven and woven fabrics of natural and synthetic fibers may be used as a fabric sheet in the present invention. Specific examples of the nonwoven and woven fabrics include those made from materials such as rayon, acetate, acrylate, polyester, polyethylene, polypropylene, polyurethane, polyamide, cotton and blend fibers thereof. Additional examples include wet-process and dry-process pulp sheets, and pulp sheets reinforced by thermoplastic resins (such as polyethylene, polypropylene and polyethylene terephthalate). Preferably, these sheets have a short inter-fiber distance so that the powder exist in the vicinity of the sheet surface. Pulp and cotton are particularly preferred sheet materials because of ease in processability in fabrication. Using a stack of thin pulp layers is preferred in order to obtain both softness and strength of sheet. Thermoplastic resin-reinforced pulp is also preferred because such pulp gives an appropriate thickness (bulkiness) by embossing and does not excessively soak the skin. In addition, a sheet comprising a plurality of sheets partly combined to each other by heat seal leads to a product which is soft and thick enough to make a satisfactory wiping, thereby providing a comfortable feeling when the sheet is used.

[Dynamic friction resistance]

If the cosmetic sheet of the present invention is used for wiping the skin, such as face, neck, hands and legs, a sufficient amount of powder remains on the skin and the dynamic friction resistance of the skin is reduced. As a result, the user of the cosmetic sheet can surely enjoy the non-sticky feeling on the skin. In order to ensure the effect of the present invention, desirably the cosmetic sheet reduces the dynamic friction resistance of the skin area after the use of the cosmetic sheet thereon to 95% or less, in particular to 80% or less, of that before application. The dynamic friction resistance and also the relative value thereof were measured in accordance with the following method.

〈Method for evaluating dynamic friction resistance〉

As a material corresponding to the human skin, an acrylonitrile/butadiene rubber (hereinafter abbreviated as NBR) mat [e.g., JIS K6380-compliance Kureseed (manufactured by Kureha Elastomer Co., Ltd., MB265N model, 1 mm thick)] is used, and a cosmetic sheet is applied to the NBR mat. The dynamic friction resistance is measured by means of a surface property tester (HEIDON-14 D model). The surface of the NBR mat is cleaned with ethanol prior to the test. The cleaned NBR mat cut in the size of 5 cm ×12 cm is fixed to the surface of a table by using a double-sided adhesive tape, a cosmetic sheet cut in the size of 5 cm×7 cm is placed thereon, and a weight of 200 g, whose bottom face is in the shape of a circle having a diameter of 5 cm, is placed on the cosmetic sheet. Under this weight, the cosmetic sheet is moved once on the NBR mat at a speed of 5 cm/second to carry out a wiping operation. After that, the NBR mat is subjected to the measurement of dynamic friction resistance by using the surface tester. That is, a clean NBR mat in the size of 3 cm×3 cm is fixed to the upper arm of the tester, while the NBR mat after wiping is fixed to the lower base of the tester. The friction resistance between the above-mentioned two mats is measured under the condition: the weight imposed by the arm is 200 g; the moving distance in horizontal direction is 7 cm; and the moving speed is 5 cm/second. The ratio between the values of dynamic friction resistance before and after wiping is used for the evaluation of the change in the dynamic friction resistance by the application of the cosmetic sheet. The ratio of the value after application to that before application are shown in the following tables as the relative dynamic friction resistance.

[Surface energy]

Among the characteristics of the powder, surface energy influences adherence and retention of the powder on the skin. To obtain a desirable result, the surface energy is preferably 70 mN/m or less, more preferably 60 mN/m or less and most preferably 40 mN/m or less. The surface energy of the above-mentioned range is also preferable for evaporation and dissipation of water which is affected by water-repellent property of the powder. In the present invention, the surface energy is measured in accordance with the following method.

〈Method for measuring the surface energy〉

Surface tension $\gamma$ is supposed to be the sum of the term ($\gamma^d$) due to London dispersion force and the term ($\gamma^p$) due to other polar force.

$$\gamma = \gamma^d + \gamma^p \tag{1}$$

The $\gamma^d$ and $\gamma^p$ of the surface of a solid can be calculated by measuring contact angles thereof with two liquids (distilled water and methylene iodide) whose $\gamma^d$ and $\gamma^p$ are known respectively.

$$(b_1 + c_1 + a_1)\gamma^d \gamma^p + c_1(b_1 - a_1)\gamma^d + b_1(c_1 - a_1)\gamma^p - a_1 b_1 c_1 = 0 \tag{2}$$

$$(b_2 + c_2 + a_2)\gamma^d \gamma^p + c_2(b_2 - a_2)\gamma^d + b_2(c_2 - a_2)\gamma^p - a_2 b_2 c_2 = 0 \tag{3}$$

$$a_1 = (1/4)\gamma_1(1 + \cos\theta_1) \qquad a_2 = (1/4)\gamma_2(1 + \cos\theta_2)$$

$$b_1 = \gamma_1^d \qquad b_2 = \gamma_2^d$$

$$c_1 = \gamma_1^p \qquad c_2 = \gamma_2^p$$

$\theta_1$; contact angle of distilled water with the surface of the solid
$\theta_2$; contact angle of methylene iodide with the surface of the solid

•Surface tension of distilled water

$\gamma_1 = 72.8$ dyne/cm
$\gamma_1^d = 22.1$ dyne/cm
$\gamma_1^p = 50.7$ dyne/cm

•Surface tension of methylene iodide

$\gamma_2 = 50.8$ dyne/cm
$\gamma_2^d = 44.1$ dyne/cm
$\gamma_2^p = 6.7$ dyne/cm

From equations (2) and (3), the dispersion force component ($\gamma^d$) and the polar force component ($\gamma^p$) of the surface tension of a solid are obtained by calculation. The surface tension, i.e., surface energy, of the solid is calculated from the equation (1) by substitution.

[Oily substance]

In order to further improve a characteristic feeling, the cosmetic sheet of the present invention may contain a silicone oil having a viscosity in the range of from 1 to 200 mm$^2$/s at 25°C. The incorporation of the silicone oil brings about a desirable effect on further improving the adherence of the powder to the skin. Examples of the silicone oil include methylpolysiloxane, dimethylpolysiloxane, diethylpolysiloxane, methylphenylpolysiloxane, fatty acid-modified polysiloxane, fatty alcohol-modified polysiloxane, and amino-modified polysiloxane. Among them, methylpolysiloxane and dimethylpolysiloxane are particularly preferred. The amount of the silicone oil to be incorporated into the cosmetic sheet is preferably from a hundredth to 10-fold amount of the powder and more preferably from a fiftieth to equal amount of the powder based on weight.

Besides the silicone oil, other oily substance may be used, in order to further improve a characteristic feeling. Preferred examples thereof include avocado oil, camellia oil, turtle oil, corn oil, olive oil, wheat germ oil, soybean oil, jojoba oil, peanut oil, cacao oil, lanolin, liquid paraffin, squalane, vaseline and cholesteryl esters.

[Other components]

Alcohols, such as ethanol, propylene glycol, 1,3-butanediol, glycerin, sorbitol and polyethylene glycol may also be incorporated into the cosmetic sheet of the present invention.

In addition, a germicide, an antiperspirant, a refreshing agent, a moisturizer, an anti-inflammatory agent, a whitening agent, a UV care agent and a perfume may also be incorporated into the cosmetic sheet of the present invention. Generally, most of these components are lipophilic and they exhibit their functional effects after being absorbed into the skin. If the powder used in the present invention is one which has a smaller surface energy and accordingly a high lipophilicity, the effective component mentioned above is adsorbed on the powder remains on the skin together with the powder. Therefore, if the skin is wiped with a cosmetic sheet having the above-mentioned composition, the skin is cleansed and a non-sticky feeling is given to the skin for a long period of time because the powder and the effective component are efficiently retained on the skin. Further, it is possible to functionally condition the skin by the use of the cosmetic sheet and to enjoy a wet and cool feeling and fragrance as well.

For example, if the cosmetic sheet is used on face, the cosmetic sheet effectively removes sebum and produces a refreshed feeling by leaving powder and the moisturizer on the face. In addition, the adjustment of makeup is made easier. In the case where an antiperspirant and a germicide are incorporated into the cosmetic sheet, a continuous antiperspirant and deodorant effect can be expected because these effective components are retained on the skin for a long period of time after the skin is cleansed. If the cosmetic sheet of the present invention is used for shaving unwanted hair, the skin is effectively conditioned before and after shaving. As a result, the skin is not damaged because the razor movement becomes smooth and a comfortable feeling of the skin can be obtained after shaving.

[Process for preparing the cosmetic sheet]

It is preferable that the powder in the cosmetic sheet of the present invention be held such that the powder can be transferred onto the skin. Further, preferably most of the powder is localized in the vicinity of the surface of the sheet. For the purpose of localizing the powder partially, for example, the powder is dusted directly over a fabric sheet or a liquid containing the powder is applied on a fabric sheet.

The cosmetic sheet of the present invention can be prepared by, for example, a process comprising the steps of spraying, coating, dipping or impregnating a sheet with a liquid composition containing at least one powder and other components dispersed in an aqueous medium such as water and water-containing ethanol so that the liquid composition is held in the fabric sheet.

More in detail, said process can be carried out by spraying the sheet with the liquid composition using a spray, an air gun, or the like; by coating the sheet with the liquid composition using a slit-type nozzle or a bar coater; or dipping the sheet into the liquid composition.

For the preparation of a stacked-cosmetic sheet, a preferred preparing process comprises the steps of providing a fabric sheet with a liquid composition containing at least one powder and an aqueous medium so that the powder and the liquid composition are held in the fabric sheet, cutting the sheet, and stacking the cut sheets. This process makes it possible to distribute the powder uniformly on the fabric sheet and to obtain a product which fully exhibits the effect of the present invention.

〈Advantageous effects of the invention〉

The use of the cosmetic sheet of the present invention cleanses the sweated or dirty skin. When the cosmetic sheet of the present invention is applied on the skin and the powder is transferred onto the skin, the dynamic friction resistance of the skin can be reduced. After the use, a non-sticky and slippery feeling is produced on the skin. As the result, a new refreshed feeling unattained by a conventional wet sheet can be given to the skin.

Examples

Examples 1-11 and Comparative Example 1

Aqueous liquid components shown in Table 1 were prepared using a homogenizing mixer. Each composition was sprayed onto the fabric sheet at respective impregnation ratios so that at least one powder was held in the sheets. Then the sheets were cut into a suitable size and the cut sheets were stacked to give a wet cosmetic sheet product which is packed in a container.

In the table, the figures given in brackets for each powder indicate surface energy and particle size, wherein the particle size is an average particle diameter (the same applies to the examples below). Of the materials listed in Table 1, Polymer Particle X were synthesized according to the following process.

〈Synthesis of Polymer Particle X〉

270 g of n-hexane, 90 g of toluene, and 2 g of a polysiloxane compound having a terminal methacryloyloxy group (Sylaprene FM-0725 (registered trademark) manufactured by Chisso Corp. were charged into a reaction vessel under a nitrogen atmosphere, and the mixture was heated to 70°C. Then, 39 g of methyl methacrylate, 13 g of styrene, and 1 g of lauroyl peroxide were added and the mixture was allowed to react at 70°C for 12 hours.

After cooling, the reaction solution was diluted with 200 g of n-hexane. The powder residue was centrifugally separated from the liquid, followded by washing twice with 300 g each of n-hexane. The solids thus obtained were dried at 50°C under vacuum in a dryer to give 54 g of a white powder. The polymer beads thus obtained were monodispersed particles having a diameter of 2 $\mu$m.

Table 1

| | | Example | | | | | | | | | | | Comparative Example |
|---|---|---|---|---|---|---|---|---|---|---|---|---|---|
| | | 1 | 2 | 3 | 4 | 5 | 6 | 7 | 8 | 9 | 10 | 11 | 1 |
| Fabric sheet | Cotton sheet (40g/m²) | ○ | | | | | ○ | | | ○ | | | ○ |
| | Cupra sheet (40g/m²) | | ○ | | | | | | ○ | | | ○ | |
| | Rayon blend sheet (40g/m²) | | | | ○ | | | | | | ○ | | |
| | Pulp sheet (40g/m²) | | | ○ | | ○ | | ○ | | | | | |
| Impregnation ratio (%) | | 300 | 250 | 200 | 450 | 150 | 330 | 250 | 300 | 200 | 250 | 270 | 300 |
| Amount of liquid composition (g/m²) | | 120 | 100 | 80 | 180 | 60 | 132 | 100 | 120 | 80 | 100 | 108 | 120 |
| Raw materials in aqueous liquid composition | Polymer Particle X (21mN/m, 2μm) | 5 | | | | | | 7 | | | | | |
| | Silicone resin (30mN/m, 3μm) | | 8 | | | | | | 3 | | | | |
| | Polystyrene resin (35mN/m, 5μm) | | | 2 | | | | | | 1 | | | |
| | Polyethylene resin (33mN/m, 7μm) | | | | 15 | | | | | | | | |
| | Nylon resin (45mN/m, 5μm) | | | | | 0.5 | | | | | | | |
| | Polymethyl methacrylate resin (39mN/m, 5μm) | | | | | | 10 | | | | | | |
| | Talc (73mN/m, 10μm) | | | | | 2 | | | 2 | 5 | 5 | | |
| | Kaolin (80mN/m, 20μm) | | | | | | | | | | | 0.08 | 20 |
| | Silicone oil (5mm²/s) | | | | | | | | | 2 | 2 | 2 | |
| | Silicone oil (20mm²/s) | | | | | | 1 | 1 | 1 | | | | |
| | Silicone oil (100mm²/s) | | | | | | | | | | | | |
| | Ethanol | 20 | 5 | | 10 | | | 15 | 5 | 5 | 20 | 20 | |
| | 1,3-butanediol | | | | | | 5 | | | | | | |
| | Carbopol ® (carboxy vinyl polymer) | | | 1 | | | | 1 | | | | | |
| | Pemulen ® (acrylic acid-alkyl methacrylate copolymer) | | | | 2 | | | | | | | | |
| | Carboxymethyl cellulose | | | | | | | | 1 | | | | |
| | Methyl para-oxybenzoate | | | 0.5 | 1 | 0.2 | 1 | 0.5 | 1 | 0.2 | | | |
| | Ethyl para-oxybenzoate | | | | | | | | 0.5 | | 0.4 | 0.4 | |
| | Perfume | | | | | | | | | | | | |
| | Water | 95 | 87 | 76.5 | 152 | 57.3 | 115 | 75.5 | 106.5 | 64.8 | 72.6 | 85.52 | 100 |
| Relative dynamic friction resistance (%) | | 74 | 72 | 76 | 70 | 79 | 78 | 69 | 75 | 80 | 80 | 94 | 110 |

Amounts of raw materials are based on g/m²

7

Test method

5 males and 5 females all aged between 20 and 40 tested the cosmetic sheets of Examples 1~11 and Comparative Example 1 by wiping respective arms and necks. They evaluated the feeling according to the following criteria. The relative dynamic friction resistance was also examined in accordance with the aforementioned method. The results are shown in Table 2. In addition, by rating the results according to the following points, average marks per cosmetic sheet by 10 persons are also shown in Table 2.

〈 Items of evaluation 〉

| Non-sticky feeling | |
|---|---|
| ○ : non-sticky | 2 |
| Δ: slightly non-sticky | 1 |
| X: sticky | 0 |
| Skin roughening | |
| ○ : skin is not roughened | 2 |
| Δ: slightly roughened | 1 |
| X: roughened | 0 |
| Wet feeling | |
| ○ : comfortable | 2 |
| X: dry feeling | 0 |
| X: excessively wet | 0 |
| White appearance after use | |
| ○ : no white appearance | 2 |
| Δ: slight white appearance | 1 |
| X: white appearance remains | 0 |
| Duration of non-sticky feeling | |
| ○ : very good | 2 |
| Δ: good | 1 |
| X: not good | 0 |

From the results shown in Table 2, the cosmetic sheets of Examples 1~11 are superior to that of Comparative Example 1 in the feeling upon use. More specifically, in the case of Examples 1~11, non-stickiness was really felt, the skin was not roughened, the powder did not turn the skin white, and the comfortable feeling lasted.

Table 2

| Test results | | | 1 | 2 | 3 | 4 | 5 | 6 | 7 | 8 | 9 | 10 | 11 | Comparative Example 1 |
|---|---|---|---|---|---|---|---|---|---|---|---|---|---|---|
| | | | | | | | | Example | | | | | | |
| | Non-sticky feeling | ◯: non-sticky | 9 | 10 | 8 | 10 | 7 | 10 | 10 | 10 | 8 | 7 | 3 | 0 |
| | | △: slightly non-sticky | 1 | 0 | 2 | 0 | 2 | 0 | 0 | 0 | 2 | 2 | 4 | 0 |
| | | ✕: sticky | 0 | 0 | 0 | 0 | 1 | 0 | 0 | 0 | 0 | 1 | 3 | 10 |
| | | Average mark | 1.9 | 2.0 | 1.8 | 2.0 | 1.6 | 2.0 | 2.0 | 2.0 | 1.8 | 1.6 | 1.0 | 0 |
| | Skin roughening | ◯: skin is not roughened | 9 | 10 | 8 | 10 | 9 | 9 | 9 | 9 | 10 | 9 | 9 | 0 |
| | | △: slightly roughened | 1 | 0 | 2 | 0 | 1 | 1 | 1 | 1 | 0 | 1 | 1 | 3 |
| | | ✕: roughened | 0 | 0 | 0 | 0 | 0 | 0 | 0 | 0 | 0 | 0 | 0 | 7 |
| | | Average mark | 1.9 | 2.0 | 1.8 | 2.0 | 1.9 | 1.9 | 1.9 | 1.9 | 2.0 | 1.9 | 1.9 | 0.3 |
| | Wet feeling | ◯: comfortable | 9 | 9 | 8 | 7 | 7 | 8 | 8 | 10 | 8 | 9 | 8 | 9 |
| | | ✕: dry feeling | 0 | 1 | 2 | 0 | 3 | 0 | 2 | 0 | 2 | 1 | 2 | 0 |
| | | ✕: excessively wet | 1 | 0 | 0 | 3 | 0 | 2 | 0 | 0 | 0 | 0 | 0 | 1 |
| | | Average mark | 1.8 | 1.8 | 1.6 | 1.4 | 1.4 | 1.6 | 1.6 | 2.0 | 1.6 | 1.8 | 1.6 | 1.8 |
| | White appearance after use | ◯: no white appearance | 10 | 9 | 10 | 8 | 10 | 9 | 10 | 8 | 10 | 7 | 9 | 10 |
| | | △: slight white appearance | 0 | 1 | 0 | 2 | 0 | 1 | 0 | 2 | 0 | 3 | 1 | 0 |
| | | ✕: white appearance | 0 | 0 | 0 | 0 | 0 | 0 | 0 | 0 | 0 | 0 | 0 | 0 |
| | | Average mark | 2.0 | 1.9 | 2.0 | 1.8 | 2.0 | 1.9 | 2.0 | 1.8 | 2.0 | 1.7 | 1.9 | 2.0 |
| | Duration of non-sticky feeling | ◯: very good | 8 | 8 | 7 | 8 | 6 | 7 | 10 | 10 | 8 | 6 | 2 | 0 |
| | | △: good | 2 | 2 | 3 | 2 | 3 | 2 | 0 | 0 | 1 | 3 | 5 | 0 |
| | | ✕: not good | 0 | 0 | 0 | 0 | 1 | 1 | 0 | 0 | 1 | 2 | 3 | 10 |
| | | Average mark | 1.8 | 1.8 | 1.7 | 1.8 | 1.5 | 1.6 | 2.0 | 2.0 | 1.7 | 1.5 | 0.9 | 0 |

EP 0 950 391 A1

Examples 12~16 and Comparative Example 2

Aqueous liquid components shown in Table 3 were prepared using a homogenizing mixer. Each composition was coated onto the fabric sheet by using a slit-type nozzle at respective impregnation ratios, so that at least one powder was held in the sheets. Then the sheets were cut into a suitable size and the cut sheets were stacked to give a wet cosmetic sheet product packed in a container. 10 females aged between 20 and 40 tested the cosmetic sheets by wiping respective faces. They evaluated the cosmetic sheets with regard to the items shown in Table 3. The items "non-sticky feeling" and "skin roughening" were rated as described in Table 2. In addition, "stickiness-free feeling on face" and "ease in applying foundation after using cosmetic sheet" were rated according to the following criteria. Average marks by the 10 persons are listed in Table 3.

〈 Item of evaluation〉

Stickiness-free feeling on face

2:     non-sticky
1:     slightly sticky
0:     sticky

Ease in applying foundation after using cosmetic sheet

2:     very good
1:     good
0:     not good

From the results shown in Table 3, the cosmetic sheets of Examples 12~16 are superior to that of Comparative Example 2. More specifically, the cosmetic sheets of Examples 12~16 keep the face in a refreshed state after removing sebum and stickiness from face, do not roughen the skin, and facilitate further application of foundation.

Table 3

| | | | Example | | | | | Comparative Example |
|---|---|---|---|---|---|---|---|---|
| | | | 12 | 13 | 14 | 15 | 16 | 2 |
| Fabric sheet | | Cotton sheet (40g/m²) | ○ | | | | | ○ |
| | | Cupra sheet (50g/m²) | | | | | ○ | |
| | | Rayon blend sheet (40g/m²) | | | | ○ | | |
| | | Pulp sheet (30g/m² × 2PLY, embossed) | | ○ | ○ | | | |
| | | Impregnation ratio (%) | 270 | 250 | 200 | 150 | 400 | 300 |
| | | Amount of liquid composition (g/m²) | 108 | 150 | 120 | 90 | 200 | 120 |
| Raw materials in aqueous liquid composition | | Polymer Particle X (21mN/m,2μm) | 2 | | | | | |
| | | Silicone resin (30mN/m,3μm) | | 2 | | | | |
| | | Polystyrene resin (35mN/m,5μm) | | | 0.5 | | | |
| | | Polyethylene resin (33mN/m,7μm) | | 2 | | | | |
| | | Nylon resin (45mN/m,6μm) | | | | 10 | | |
| | | Polymethyl methacrylate resin (39mN/m,5μm) | | | | | 10 | |
| | | Talc (73mN/m,10μm) | | 1 | | | 4 | |
| | | Kaolin (80mN/m,20μm) | | | 2 | | | |
| | | Silicone oil (5mm²/s) | 2 | | | 1 | | |
| | | Silicone oil (20mm²/s) | | 1 | | | | |
| | | Silicone oil (100mm²/s) | | | | | 5 | |
| | | Ethanol | 15 | 5 | | 10 | 12 | 15 |
| | | 1,3-butanediol | 2 | | 5 | | | 2 |
| | | Carbopol ® (carboxy vinyl polymer) | 0.3 | | 1 | | | |
| | | Pemulen ® (acrylic acid-alkyl methacrylate copolymer) | | 0.5 | | | 1 | |
| | | Carboxymethyl cellulose | | | | 1 | | |
| | | Methyl para-oxybenzoate | | 0.3 | 0.2 | | 1 | |
| | | Ethyl para-oxybenzoate | 0.1 | | | 0.1 | | |
| | | Perfume | | | 0.1 | | | |
| | | Water | 86.6 | 138.2 | 111.2 | 67.9 | 167 | 103 |
| | | Relative dynamic friction resistance (%) | 76 | 75 | 79 | 72 | 74 | 111 |
| Test results of the feeling (average mark) | | Non-sticky feeling | 1.9 | 1.8 | 1.6 | 1.9 | 1.8 | 0 |
| | | Stickiness-free feeling on face | 1.8 | 1.6 | 1.6 | 1.8 | 1.6 | 1.2 |
| | | Skin roughening | 1.7 | 1.9 | 1.8 | 1.7 | 2.0 | 0.2 |
| | | Ease in applying foundation after using cosmetic sheet | 1.8 | 1.7 | 1.7 | 1.8 | 1.8 | 0.2 |

Amounts of raw materials are based on g/m²

Examples 17~21 and Comparative Example 3

5 males and 5 females all aged between 20 and 40 tested the cosmetic sheets listed in Table 4 by cleansing respective armpits. They evaluated the cosmetic sheets with regard to the items shown in Table 4. The items "non-sticky feeling" and "skin roughening" were rated as described in Table 2. In addition, "smell after using cosmetic sheet" and "smell of skin 6 hours after using cosmetic sheet" were rated according to the following criteria. Average marks by the 10 persons are listed in Table 4.

Smell after using cosmetic sheet

〈Items of evaluation〉

2:     no smell
1:     slight smell
0:     smelly

Smell of skin 6 hours after using cosmetic sheet

2:     no smell
1:     slight smell
0:     smelly

From the results shown in Table 4, the cosmetic sheets of Examples 17~21 are superior to that of Comparative Example 3. More specifically, the cosmetic sheets of Examples 17~21 surely remove the smell from armpits, keep the armpits in a comfortable state after using, do not roughen the skin, and inhibit the smell of armpits for a long period of time.

Table 4

| | | Example | | | | | Comparative Example |
|---|---|---|---|---|---|---|---|
| | | 17 | 18 | 19 | 20 | 21 | 3 |
| Fabric sheet | Cotton sheet (60g/m²) | ○ | | | | | ○ |
| | Cupra sheet (50g/m²) | | | ○ | | | |
| | Rayon blend sheet (80g/m²) | | | | ○ | | |
| | Pulp sheet (50g/m²) | | ○ | | | ○ | |
| | Impregnation ratio (%) | 240 | 280 | 250 | 120 | 180 | 250 |
| | Amount of liquid composition (g/m²) | 144 | 140 | 125 | 96 | 90 | 150 |
| Raw materials in aqueous liquid composition | Polymer Particle X (21mN/m,2μm) | 7 | | 0.5 | | | |
| | Silicone resin (30mN/m,3μm) | | | | | 2 | |
| | Polystyrene resin (35mN/m,5μm) | | 5 | | | | |
| | Polyethylene resin (33mN/m,7μm) | | 2 | | | | |
| | Nylon resin (45mN/m,6μm) | | | 0.5 | | | |
| | Polymethyl methacrylate resin (39mN/m,5μm) | | | | 4 | | |
| | Talc (73mN/m,10μm) | | | | 10 | 2 | |
| | Aluminum hydroxychloride | | 5 | | | 2 | |
| | Isopropylmethylphenol | 2 | | 1 | | | |
| | Trichlorocarbanilide | | 2 | | | | |
| | Trichlosan | | | | 1 | | |
| | Silicone oil (5mm²/s) | | 5 | | 0.5 | 2 | |
| | Silicone oil (20mm²/s) | | | 2 | | | |
| | Ethanol | 20 | | 15 | 10 | 10 | 30 |
| | Propylene glycol | | 10 | | | 2 | 5 |
| | Carbopol ® (carboxy vinyl polymer) | | 1 | | | 0.4 | |
| | Pemulen ® (acrylic acid-alkyl methacrylate copolymer) | 0.5 | | | 1 | | |
| | Carboxymethyl cellulose | | | 1 | | | |
| | Methyl para-oxybenzoate | 0.1 | | 0.6 | | | |
| | Ethyl para-oxybenzoate | | 0.2 | | 0.1 | 0.2 | |
| | Perfume | | 0.05 | 0.2 | | | |
| | Water | 114.4 | 109.75 | 104.2 | 69.4 | 69.4 | 115 |
| | Relative dynamic friction resistance (%) | 69 | 76 | 79 | 78 | 71 | 109 |
| Test results of the feeling (average mark) | Non-sticky feeling | 1.9 | 1.8 | 1.6 | 1.9 | 1.8 | 0.1 |
| | Skin roughening | 1.8 | 1.9 | 2.0 | 1.8 | 1.9 | 0.3 |
| | Smell after using cosmetic sheet | 2.0 | 1.6 | 2.0 | 1.9 | 1.9 | 1.3 |
| | Smell of skin 6 hours after using cosmetic sheet | 1.8 | 1.6 | 1.4 | 1.5 | 1.5 | 0.2 |

Amounts of raw materials are based on g/m²

Examples 22~26 and Comparative Example 4

10 females aged between 20 and 40 tested the cosmetic sheets listed in Table 5. They wiped their legs and armpits having unwanted hair with the cosmetic sheets and thereafter removing the unwanted hair by using a razor. Evaluation were made with regard to the items shown in Table 5. Average marks by the 10 persons are listed in Table 5.

〈Items of evaluation〉

Feeling of skin after using cosmetic sheet

2:　　non-sticky feeling
1:　　slightly non-sticky
0:　　sticky

White appearance after using cosmetic sheet

2:　　no white appearance
1:　　slight white appearance
0:　　white appearance remains

From the results shown in Table 5, the cosmetic sheets of Examples 22~26 are superior to that of Comparative Example 4. More specifically, the cosmetic sheets of Examples 22~26 produce a more comfortable feeling, do not turn the skin white and make the skin feel comfortable after using the cosmetic sheets.

# EP 0 950 391 A1

Table 5

| | | Example | | | | | Comparative Example |
|---|---|---|---|---|---|---|---|
| | | 22 | 23 | 24 | 25 | 26 | 4 |
| Fabric sheet | Cotton sheet (40g/m²) | ○ | | | ○ | | ○ |
| | Cupra sheet (40g/m²) | | | ○ | | | |
| | Rayon blend sheet (40g/m²) | | | | | ○ | |
| | Pulp sheet (40g/m²) | | ○ | | | | |
| Impregnation ratio (%) | | 300 | 200 | 250 | 350 | 400 | 250 |
| Amount of liquid composition (g/m²) | | 120 | 80 | 100 | 140 | 160 | 100 |
| Raw materials in aqueous liquid composition | Polymer Particle X (21mN/m,2μm) | 3 | | | | | |
| | Silicone resin (30mN/m,3μm) | | | | 4 | | |
| | Polystyrene resin (35mN/m,5μm) | | 0.5 | | | | |
| | Polyethylene resin (33mN/m,7μm) | | | | | 6 | |
| | Nylon resin (45mN/m,6μm) | | | 1 | | | |
| | Talc (73mN/m,10μm) | 3 | | 5 | 7 | | |
| | Kaolin (80mN/m,20μm) | | 4 | | | 2 | |
| | Silicone oil (5mm²/s) | 1 | | | 5 | 2 | |
| | Silicone oil (20mm²/s) | | 3 | 2 | | | |
| | Ethanol | 15 | | 5 | 15 | | 10 |
| | Propylene glycol | | 5 | | | 10 | |
| | Carbopol ® (carboxy vinyl polymer) | | 0.1 | | | 0.1 | |
| | Pemulen ® (acrylic acid-alkyl methacrylate copolymer) | 0.1 | | | 0.2 | | |
| | Carboxymethyl cellulose | | | 0.1 | | | |
| | Methyl para-oxybenzoate | 0.1 | | 0.2 | | | |
| | Ethyl para-oxybenzoate | | 0.2 | | 0.3 | 0.2 | |
| | Perfume | | | 0.2 | | | |
| | Water | 97.8 | 67.2 | 86.5 | 108.5 | 139.7 | 90 |
| | Relative dynamic friction resistance (%) | 70 | 78 | 76 | 72 | 74 | 107 |
| Test results of the feeling (average mark) | Feeling of skin after using cosmetic sheet | 1.8 | 1.9 | 1.7 | 1.9 | 1.6 | 0 |
| | White appearance after using cosmetic sheet | 1.9 | 2.0 | 2.0 | 1.8 | 1.6 | 2.0 |

Amounts of raw materials are based on g/m²

## Claims

1.  A cosmetic sheet comprising at least one powder selected from a synthetic polymer and a natural mineral and an aqueous medium held together in a fabric sheet.

2.  The cosmetic sheet according to Claim 1, which is applied to the human skin and reduces the skin's dynamic friction resistance to 95% or less of that before application.

3.  The cosmetic sheet according to Claim 1, wherein the powder is selected from a synthetic polymer.

15

4. A cosmetic sheet according to Claim 1, wherein a surface energy of the powder is 70mN/m or less.

5. The cosmetic sheet according to Claim 1, wherein the powder is composed of polymer containing organosilicone groups.

6. The cosmetic sheet according to Claim 1, which contains a silicone oil having a viscosity of 1 to 200 mm$^2$/s at 25°C in an amount of a hundredth to 10-fold of the powder based on weight.

7. A method for reducing the dynamic friction resistance of the human skin by applying the cosmetic sheet of Claim 1 on the skin so that the powder is transferred onto the skin.

8. A process for preparing a stacked cosmetic sheet product, comprising the steps of (A) providing a fabric sheet with a liquid composition containing at least one powder and an aqueous medium so that the liquid composition is held in the fabric sheet, (B) cutting the sheet, and (C) stacking the cut sheets.

9. The cosmetic sheet according to Claim 1, wherein the fabric sheet is a nonwoven fabric or a woven fabric.

# EUROPEAN SEARCH REPORT

**European Patent Office**

**Application Number**

EP 99 10 6278

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | DE 27 30 266 A (COLLO) 25 January 1979 (1979-01-25) * claims 1-3,6,7,10,11 * | 1,7,9 | A61K7/00 |
| X | EP 0 104 679 A (UNILEVER) 4 April 1984 (1984-04-04) * claims 1,2,5,26 * * page 3, line 24-34 * | 1,7 | |
| P,X | EP 0 870 498 A (L'OREAL) 14 October 1998 (1998-10-14) * claims 1,3-5,7,13,19,20 * | 1,3,9 | |
| | | | **TECHNICAL FIELDS SEARCHED (Int.Cl.6)** |
| | | | A61K |

The present search report has been drawn up for all claims

| Place of search | Date of completion of the search | Examiner |
|---|---|---|
| THE HAGUE | 12 July 1999 | Peeters, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03 82 (P04C01)

**ANNEX TO THE EUROPEAN SEARCH REPORT
ON EUROPEAN PATENT APPLICATION NO.**                EP 99 10 6278

This annex lists the patent family members relating to the patent documents cited in the above-mentioned European search report.
The members are as contained in the European Patent Office EDP file on
The European Patent Office is in no way liable for these particulars which are merely given for the purpose of information.

12-07-1999

| Patent document cited in search report | | Publication date | Patent family member(s) | | Publication date |
|---|---|---|---|---|---|
| DE 2730266 | A | 25-01-1979 | NONE | | |
| EP 104679 | A | 04-04-1984 | AT | 45378 T | 15-08-1989 |
| | | | AU | 543153 B | 04-04-1985 |
| | | | AU | 1853483 A | 12-04-1984 |
| | | | BR | 8304746 A | 10-04-1984 |
| | | | FI | 833088 A | 02-03-1984 |
| | | | GB | 2126600 A,B | 28-03-1984 |
| | | | GR | 79625 A | 31-10-1984 |
| | | | IN | 157283 A | 22-02-1986 |
| | | | JP | 1292682 C | 16-12-1985 |
| | | | JP | 59059796 A | 05-04-1984 |
| | | | JP | 60015680 B | 20-04-1985 |
| | | | PT | 77274 A,B | 01-09-1983 |
| | | | TR | 22424 A | 18-05-1987 |
| | | | US | 4537604 A | 27-08-1985 |
| | | | ZA | 8306457 A | 24-04-1985 |
| | | | ZW | 18783 A | 10-04-1985 |
| EP 870498 | A | 14-10-1998 | FR | 2761889 A | 16-10-1998 |
| | | | CA | 2232616 A | 11-10-1998 |
| | | | JP | 2865659 B | 08-03-1999 |
| | | | JP | 10287559 A | 27-10-1998 |

EPO FORM P0459

For more details about this annex : see Official Journal of the European Patent Office, No. 12/82